# FASCICULE DE BREVET EUROPEEN

(11) **EP 1 659 398 B1**
(45) Date de publication et mention de la délivrance du brevet: **30.06.2010**
(21) Numéro de dépôt: 05292431.3
(22) Date de dépôt: 16.11.2005
(51) Int. Cl.: G01N 27/447, C07K 1/26

(54) **Procédé d'analyse de l'hémoglobine par électrophorèse capillaire, trousse pour électrophorèse capillaire et utilisation de ralentisseur de flux dans un tel procédé**
Procedure for analysing haemoglobin by means of capillary electrophoresis, kit for capillary electrophoresis and use of flow retarder in such a procedure
Verfahren zur Analyse von Hämoglobin durch Kapillarelektrophorese, Besteck für die Kapillarelektrophorese und Verwendung eines Strömungsverlangsamers in einem solchen Verfahren

(30) Priorité: 18.11.2004 FR 0412263
(43) Date de publication de la demande: 24.05.2006
(73) Titulaire: SEBIA, 91090 Lisses (FR)
(72) Inventeur: Robert, Frédéric, 91540 Mennecy (FR); Clement, Jean-Baptiste, 91450 Etiolles (FR); Simonin, Denis, 91000 Evry (FR)
(74) Mandataire: Vaillant, Jeanne

(56) Documents cités:
- WO-A-97/04308
- FR-A- 1 554 045
- US-A- 5 447 612
- US-A- 5 536 382
- US-A- 5 599 433

## Description

La présente invention concerne un procédé de séparation de l'hémoglobine par électrophorèse capillaire ainsi que des compositions de tampon utiles pour cette séparation, et des trousses d'analyse de l'hémoglobine par électrophorèse capillaire.

Pour analyser l'hémoglobine A₂ et les variants de l'hémoglobine, notamment, dans des liquides biologiques, comme du sang, en ayant des visées analytiques et notamment diagnostiques, et pour donc séparer des hémoglobines par électrophorèse, il est connu d'utiliser des techniques d'électrophorèse capillaire (EC). Par hémoglobines, on entend ici toute hémoglobine normale ou pas, et les variants de ces hémoglobines. L'un des intérêts de l'électrophorèse capillaire réside dans le fait que seules de très petites quantités des liquides biologiques à analyser sont nécessaires. De plus, la séparation par cette technique peut être très rapide, dans la mesure où de forts voltages peuvent être utilisés sans que l'échantillon ne s'échauffe trop lors de la séparation.

Ainsi, la technique de choix est l'analyse par isoélectrofocalisation capillaire (clEF). Cette méthode permet d'obtenir une résolution élevée entre les différentes formes d'hémoglobine (Hempe) (7). Toutefois son automatisation est difficile et, la nécessité d'utiliser des capillaires revêtus, encore appelés capillaires « coatés » afin de supprimer le flux électro-osmotique, la rend difficilement compatible avec des analyses effectuées en série.

Une autre technique dite technique de « double coating dynamique » peut être mise en oeuvre notamment avec les trousses commercialisées sous le nom de « Analis HbA₂-CE kit » ou « CEofix HbA₂₋CE kit» d'Analis. Cette technique implique un premier lavage du capillaire par une solution contenant un polycation à un pH de 4,7, suivi d'un second lavage avec un tampon d'analyse contenant un polyanion, à un pH de 8,7. Dans cette méthode de « double-coating » ou double revêtement, la quantité de charges négatives présentes sur la paroi interne du capillaire est encore plus élevée que sur un capillaire nu, si bien que le flux électro-osmotique est encore plus important. Cette méthode de double-coating dynamique ne permet toutefois pas la séparation suffisante entre les fractions HbA₂, HbC et HbE. Ceci rend l'analyse quantitative de la fraction HbA₂ impossible en présence de variants HbC ou HbE. De plus, comme les fractions HbS et HbD ont la même position électrophorétique, une analyse complémentaire en milieu acide est nécessaire afin de déterminer le type de variant présent dans l'échantillon. Enfin, le revêtement double doit être refait entre chaque analyse d'échantillon, ce qui rend cette méthode lourde et difficile à employer pour des essais en grande série.

Selon WO 97/04308, en électrochromatographie, des polyamines libres sont utilisées pour la séparation d'énantiomères. Par ailleurs, US 5 447 612 décrit que l'on peut utiliser certains systèmes tampon particuliers pour maintenir les gradients de pH requis en isoélectrofocalisation.

Par ailleurs, des séparations des hémoglobines en solution libre ont été décrites, mais elles ne répondent pas aux critères de précision, de résolution ou de rapidité attendus pour rationaliser les analyses des hémoglobines en EC. US 5 536 382 propose une méthode d'analyse des constituants d'échantillon et décrit notamment des échantillons sanguins préalablement mélangés à un réactif marqué spécifique. En FSCE, l'analyse d'une hémoglobine glyquée est décrite, le réactif marqué étant spécifique de l'hémoglobine humaine A1C. Ishoka (1)(1992) décrit la séparation d'hémoglobines en utilisant un tampon borate (100mM), à un pH de 9,98 dans des temps de migration de l'ordre de 50 minutes, c'est-à-dire incompatibles avec la durée des analyses des hémoglobines comme elles sont menées à l'heure actuelle. Le même type de tampon, à des conditions similaires de concentration et de pH (Jenkins)(3), (4) et (5) ne permet que des résolutions insuffisantes entre les fractions HbA, HbF et HbS. Sahin (2) décrit des conditions de pH plus acide, mais des résultats très insuffisants pour la résolution entre les fractions HbA, HbF, HbS et HbA₂ avec des concentrations (20mM), moindres en borate, ou avec du barbital (50mM), à un pH de 8,5, et également très insuffisants pour la résolution entre les fractions HbA, HbS et HbA₂ pour le tampon Tris (1M) à un pH de 8,0. De plus, des associations Tris/Arginine (Shihabi) (6), ont été utilisées et même si elles permettent la séparation HbA/HbS, les fractions HbC/HbE et HbA₂ ne sont pas résolues. Enfin, les brevets US 5 202 006 et US 5 439 825 qui décrivent l'utilisation du barbital ou de l'éthylbarbital ne permettent d'obtenir que de faibles résolutions entre les principales Hbs que sont les HbA, HbF, HbS et HbC.

Il subsiste donc un besoin pour une méthode d'analyse de l'hémoglobine et notamment de l'hémoglobine A₂ qui permette l'analyse en une unique étape, et sans double revêtement, qui puisse être mise en oeuvre automatiquement et en séries, et qui garantisse une résolution satisfaisante entre les formes HbA₂, HbC, HbD, HbE, HbS, HbF et HbA, notamment.

La demanderesse a maintenant mis en évidence qu'en utilisant un tampon d'analyse zwitterionique associé à un ralentisseur de flux, notamment en EC en solution libre, il est possible d'obtenir une séparation largement améliorée des fractions évoquées ci-dessus, en une étape unique, évitant ainsi les séparations complémentaires, et sans double revêtement, ce qui simplifie la mise en oeuvre. De préférence, c'est un procédé d'électrophorèse capillaire en solution libre qui est mis en oeuvre (FSCE pour « Free Solution Capillary Electrophoresis »).

Ainsi, la présente invention concerne la séparation par électrophorèse capillaire des hémoglobines dans des échantillons biologiques, procédé dans lequel on fait passer l'échantillon biologique comportant lesdites hémoglobines dans un capillaire contenant un tampon d'analyse, comportant au moins une étape où l'on introduit l'échantillon dans un tube capillaire contenant une solution de tampon d'analyse, et dans lequel le tampon est du type zwitterionique et est associé à au moins un ralentisseur de flux. Cette étape est en général suivie de la séparation des hémoglobines, par migration et détection des différents variants.

En tant que tampon zwitterionique, on utilise selon l'invention un tampon zwitterionique tamponnant entre les valeurs de pH 8 et 10, et comprenant au moins une fonction amine, et au moins une fonction acide, et au moins une fonction hydroxyle en position opposée à la fonction acide. Par fonction acide, on entend ici la fonction acide carboxylique ou la fonction acide sulfonique, notamment. Ce tampon zwitterionique peut être formé d'une ou de deux molécules : pour le cas où la fonction amine est portée par une première molécule sans fonction acide, cette première molécule est associée à une seconde molécule portant une fonction acide, notamment une fonction acide carboxylique ou sulfonique ou telle qu'un acide aminé. On peut citer, à titre d'exemple, la glycine comme acide aminé.

Selon l'invention, les ralentisseurs de flux sont du type diamine ou polyamine aliphatique ou cyclique. Ils sont choisis parmi les diamines ou polyamines aliphatiques et/ou les diamines ou polyamines cycliques, par exemple. On préfère les diamines ou polyamines aliphatiques. A titre de diamine aliphatique, on peut citer à titre d'exemple le 1,3-diaminopropane, le 1,4-diaminobutane, le 1,5-diaminopentane, le 1,6-diaminohexane, la N,N'-diméthyl-1,6-hexanediamine, la N,N,N',N'-tétraméthyl-1,4-butanediamine et leurs dérivés et sels acceptables. A titre de polyamines aliphatiques, on peut citer à titre d'exemple la diéthylènetriamine, la spermine, la tétraéthylènepentamine, et leurs dérivés et sels acceptables. Les ralentisseurs de flux peuvent être utilisés en mélange.

Comme sel acceptable, on peut citer les sels de chlorhydrate ou similaires. Comme dérivés, on peut citer par exemple, les dérivés des composés ci-dessus dont un ou des atomes de carbone de la chaîne aliphatique est ou sont substitué(s) par un ou des groupements alkyles et/ou un ou des hydrogène(s) des amines libres est ou sont substitué(s) par un ou des groupements alkyles.

La solution de tampon d'analyse peut en outre comporter d'autres additifs, notamment d'autres additifs visant à améliorer la séparation des différentes hémoglobines.

De plus, l'invention concerne l'utilisation de composés connus pour leur activité de ralentisseur de flux électrophorétique, en EC en solution libre en association avec au moins un tampon zwitterionique.

Comme cela apparaît dans les exemples qui suivent, l'utilisation des associations selon l'invention permet une résolution très améliorée de l'hémoglobine et des variants de l'hémoglobine. Elle permet de ce fait d'améliorer la justesse et la précision de l'analyse qualitative et quantitative des variants, par rapport aux analyses réalisées avec les procédés connus. Elle permet également de quantifier HbA₂, même en présence de HbC ou HbE.

Les associations tampon zwitterionique-ralentisseur de flux de l'invention sont particulièrement utiles pour l'analyse d'échantillons où les hémoglobines normales ou variants du type HbA₂, HbC, HbD, HbE, HbS, HbF et/ou HbA sont présents, pour les déceler ou les quantifier.

Enfin, l'invention a pour objet des trousses (ou kits) d'analyse par EC de l'hémoglobine A₂ et des variants de l'hémoglobine dans un échantillon biologique, comprenant au moins une solution de tampon d'analyse contenant au moins un tampon d'analyse du type zwitterionique et au moins un ralentisseur de flux, et éventuellement un modificateur de pH. Ainsi, les trousses peuvent comprendre au moins un tampon d'analyse et un ralentisseur de flux selon l'invention, et une ou des solution(s) de lavage des capillaires et/ou des barrettes de dilution et/ou un (ou des) diluant(s) de l'échantillon à analyser. Elles peuvent comporter en outre au moins une solution hémolysante. Dans ce kit, le tampon et le ou les ralentisseurs(s) et diluant(s) ou autres additifs peuvent être stocké(s) séparément pour être mélangés extemporanément, ou stockés en mélange. Ce kit comprend éventuellement, en outre, des indications d'utilisation pour réaliser l'analyse et/ou un support de logiciel d'identification.

D'autres caractéristiques et avantages de l'invention apparaîtront à la lecture de la description détaillée qui va suivre, des exemples et figures annexées.

La figure 1 représente un électrophorégramme d'un sang humain normal (HbA, HbA₂) analysé par électrophorèse capillaire en utilisant une solution de tampon selon l'invention.

Les figures 2 à 5 représentent chacune un électrophorégramme d'un sang analysé par électrophorèse capillaire en utilisant la même solution de tampon. Le sang comporte respectivement les variants suivants : fig. 2 :HbF et HbS ; fig. 3 : HbC ; fig. 4 : HbE ; fig. 5 : HbS et HbD-Los Angeles.

Les figures 6a, b, c, d représentent chacune un électrophorégramme d'un sang β-thalassémique, analysé par électrophorèse capillaire en utilisant quatre solutions de tampon différentes basées sur quatre ralentisseurs de flux différents.

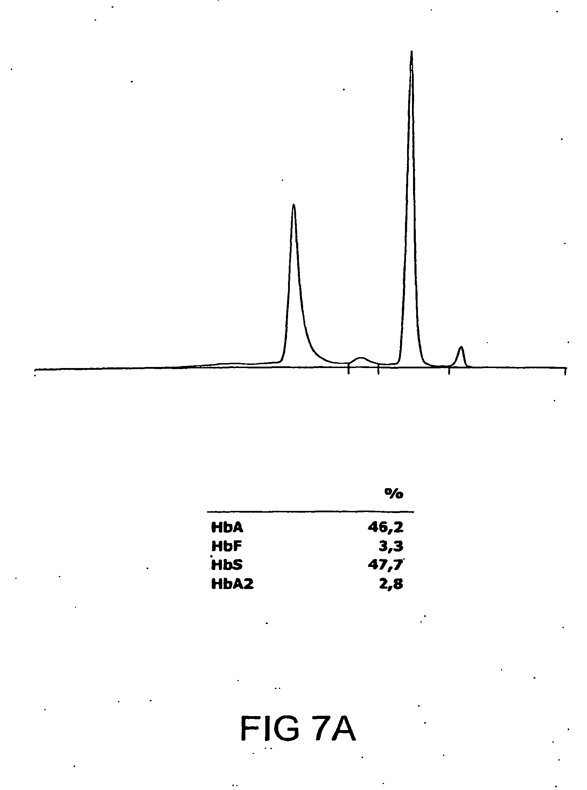

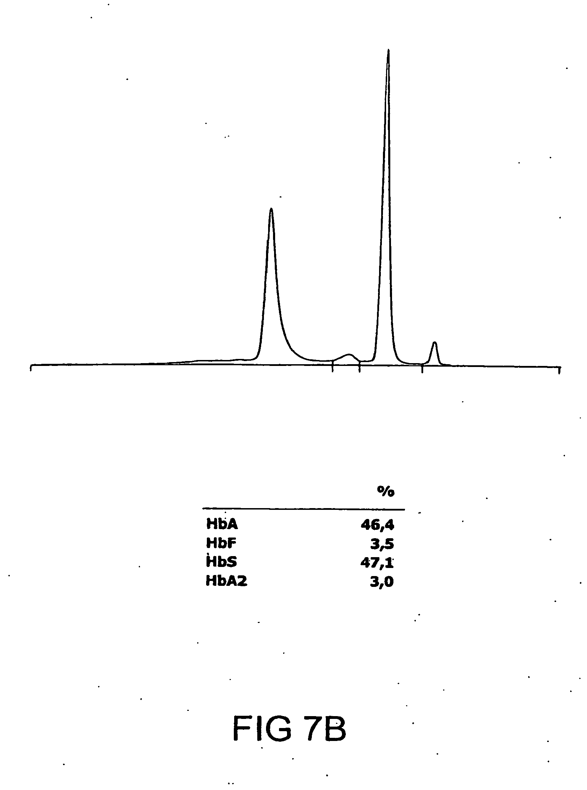

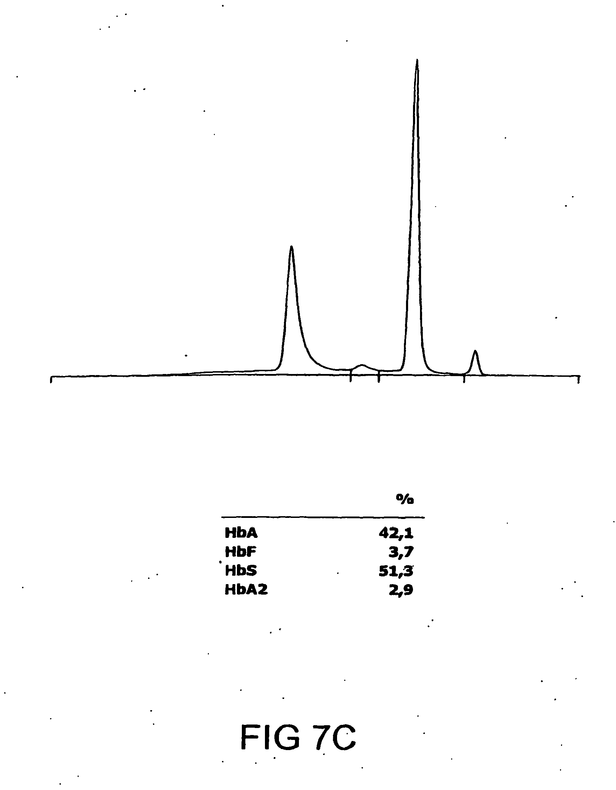

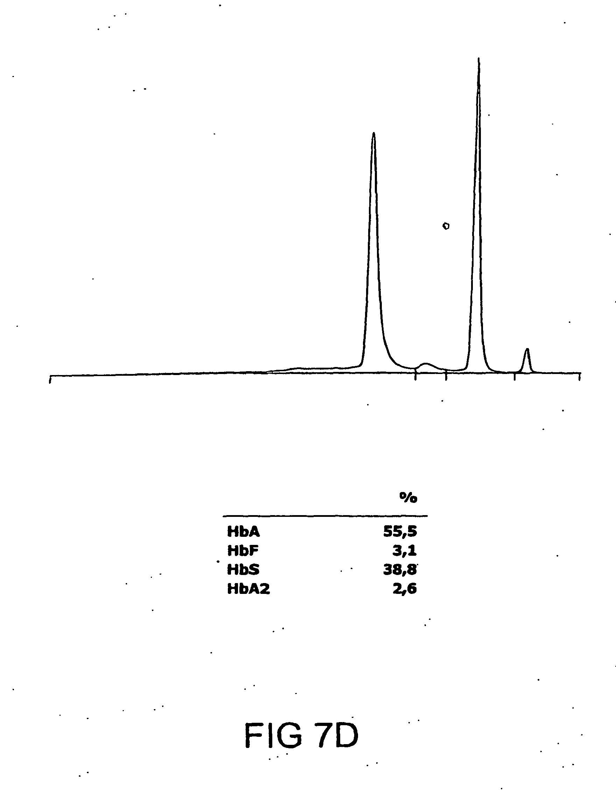

Les figures 7a, b, c, d représentent chacune un électrophorégramme d'un sang comportant HbF et HbS, analysé par électrophorèse capillaire en utilisant quatre solutions de tampon différentes basées sur quatre ralentisseurs de flux différents.

Les conditions de réalisation d'une électrophorèse capillaire EC sont connues de l'homme de l'art. Elles peuvent comporter usuellement un lavage des capillaires par une solution de lavage, un lavage avec la solution de tampon d'analyse, une ou des dilution(s) éventuelle(s) de l'échantillon, l'injection de l'échantillon, la migration et la détection. Ces étapes peuvent être réalisées par des automates.

Des conditions de réalisation d'une éléctrophorèse capillaire sont par exemple les conditions appropriées pour utiliser l'automate Capillarys (SEBIA).

A titre de tampon zwitterionique utile selon l'invention, on peut citer les tampons de type « Tris » pourvus de plusieurs groupements hydroxyle et notamment à titre d'exemples, les tampons Tris (2-amino-2-[hydroxyméthyl]-1,3-propanediol), Tricine (N-tris[hydroxyméthyl]méthylglycine), TAPS (acide N-tris[hydroxyméthyl]méthyl-3-aminopropanesulfonique), TABS (acide N-tris[hydroxyméthyl]méthyl-4-aminobutanesulfonique), ou encore AMPD (2-amino-2-méthyl-1,3-propanediol) ou Bis Tris Propane (1,3-bis[tris(Hydroxyméthyl)méthylamino]propane), ces deux derniers et le Tris devant être associés à un acide aminé.

D'autres molécules comptant un nombre de groupements hydroxyle moindre conviennent également, telles que l'AMPSO (acide 3-[(1,1-diméthyl-2-hydroxyméthyl)amino]-2-hydroxy-propanesulfonique), la Bicine (N,N-bis[2-hydroxyéthyl]glycine), l'HEPBS (acide N-[2-hydroxyéthyl]pipérazine-N'-[4-butanesulfonique]), notamment.

Parmi les tampons zwitterioniques cités ci-dessus, on préfère la tricine.

Ces tampons sont connus et disponibles dans le commerce. Ils peuvent en outre être utilisés en mélange.

Comme ralentisseur de flux, parmi ceux déjà évoqués, on préfère le 1,4-diaminobutane (DAB), le 1,5-diaminopentane, le 1,6-diaminohexane, la diéthylènetriamine (DETA) et la N,N,N',N'-tétraméthyl-1,4-butanediamine. Les ralentisseurs peuvent également être utilisés en mélange.

En association avec la tricine, on préfère le chlorhydrate de 1,4-diaminobutane.

Par échantillon selon l'invention, on entend l'échantillon biologique à analyser c'est-à-dire tout liquide biologique comportant des globules rouges provenant de patients sains ou malades. Ainsi, les liquides biologiques humains peuvent être du sang, normal ou non, lavé, décanté, centrifugé ou total. De plus, le sang peut être hémolysé.

Outre les échantillons biologiques humains, on peut analyser des échantillons d'origine animale. Les échantillons peuvent aussi être d'origine synthétique, et le procédé de l'invention peut alors avoir des visées de contrôle de production par exemple.

L'échantillon peut être préalablement dilué avec une solution de dilution appropriée, une solution hémolysante ou une solution de tampon d'analyse, par exemple.

Les procédés EC mettant en oeuvre les associations tampon zwitterionique/ralentisseur de flux selon l'invention sont particulièrement utiles pour les analyses de sang et la séparation de l'hémoglobine et de ses variants dans des échantillons humains.

Selon l'invention, le pH de la solution de tampon d'analyse est compris entre 8 et 11, de préférence entre 8 et 10.

Les solutions de tampons d'analyse selon l'invention peuvent en outre comporter au moins un composé modifiant le pH. A titre de modificateur de pH, on peut utiliser un composé choisi parmi l'hydroxyde de lithium, l'hydroxyde de sodium, l'hydroxyde de potassium, l'hydroxyde de rubidium, l'hydroxyde de césium, l'hydroxyde de francium, un hydroxyde de mono-, di-, tri- ou tétra-alkyl ammonium ayant de 1 à 8 atomes de carbone dans la partie alkyle.

Selon l'invention, les tampons sont utilisés dans les solutions de tampon d'analyse dans les conditions et concentrations usuelles, à savoir de l'ordre de 20 à 500 mM, de préférence 100 à 250 mM.

Les ralentisseurs de flux sont utilisés dans les solutions de tampon, à ces concentrations comprises entre environ 0,01 et 50 mM, de préférence environ 0,10 et 20 mM.

De plus, la solution de tampon peut comporter un ou des additifs propre(s) à modifier la force ionique.

A titre de tels composés, on peut citer des sels tels que des sulfates, des chlorures, par exemple, et leurs mélanges.

La solution hémolysante permet de réaliser l'hémolyse des globules rouges contenant l'hémoglobine A₂ et les variants de l'hémoglobine. Elle est également utile à la dilution de l'échantillon avant analyse en EC. Elle peut permettre, selon sa composition, une lyse totale des globules rouges en usant d'un faible mouvement mécanique additionnel (vortex, agitation, ...).

La solution hémolysante comprend un tampon d'analyse tels que ceux déjà évoqués (tampons zwitterioniques), des additifs usuels de lyse cellulaire, comme par exemple le Triton X100 et/ou la saponine et éventuellement d'autres additifs visant à amener un effet positif sur la résolution entre certaines hémoglobines proches. A titre d'exemple, on peut citer la solution hémolysante « Hydragel hémoglobine » de Sébia.

Le pH de la solution hémolysante est compris entre 8 et 11, de préférence entre 8 et 10.

Les solutions de tampon de l'invention sont préparées de façon usuelle pour des compositions de tampon d'analyse, à savoir par adjonction des constituants sous forme liquide, ou solide à diluer, à un support acceptable. De façon usuelle, le support est de l'eau, distillée ou déminéralisée.

Du point de vue des matériaux utilisés pour les capillaires, ceux-ci sont usuels en électrophorèse capillaire. Ainsi, on peut utiliser des capillaires de silice fondue. Leur diamètre interne peut aller de 5 à 2 000 µm. De façon préférée, on utilise selon l'invention des capillaires de diamètre interne inférieur à 200 µm, de préférence inférieur à 100 µm. On utilise de préférence des capillaires à surface intérieure non traitée. Le spécialiste saura adapter la nature du capillaire et sa taille aux besoins de l'analyse.

L'utilisation de tels capillaires nus constitue un des avantages de l'invention.

Les hémoglobines peuvent être analysées à une longueur d'onde d'environ 200 nm, sur du sang hémolysé obtenu à partir de sang lavé, décanté ou centrifugé. Néanmoins, pour éviter des interférences avec les protéines plasmatiques, elles sont de préférence analysées à une longueur d'onde d'environ 415 nm, sur sang hémolysé obtenu à partir de sang lavé, décanté, centrifugé ou total.

### Exemples

### MATERIEL ET METHODES

### A) Electrophorèse capillaire

L'électrophorèse capillaire d'échantillons cliniques est réalisée sur un appareil d'EC équipé d'un capillaire en silice fondue de diamètre interne 25 microns.

La détection est réalisée dans des conditions optimales autour de 415 nm.

Les échantillons sont placés dans l'appareil Capillarys (SEBIA) et automatiquement injectés par injection hydrodynamique. La séparation des échantillons est réalisée en moins de 8 minutes en appliquant un champ électrique d'environ 550 V/cm . Le capillaire est lavé avant chaque analyse par la soude 0,25 M, puis par la solution de tampon d'analyse.

Tampons d'analyse :

Les produits chimiques utilisés sont de grade analytique.
- Le tampon Tricine 200 mM -1,4-diaminobutane 15 mM (A) est préparé en dissolvant 35,84 g de Tricine dans environ 900 ml d'eau déminéralisée, puis en ajoutant 2,32 g de chlorhydrate de 1,4-diaminobutane (DAB). Le pH est ajusté à 9,37 à 22°C par environ 38,3 ml de NaOH 5M et le volume de la solution de tampon ajusté à 1 I par de l'eau déminéralisée.
- Le tampon Tricine 200 mM -1,5-diaminopentane 15 mM (B) est préparé en dissolvant 35,84 g de Tricine dans environ 900 ml d'eau déminéralisée, puis en ajoutant 2,62 g de chlorhydrate de 1,5-diaminopentane. Le pH est ajusté à 9,40 à 22°C par environ 38,4 ml de NaOH 5M et le volume de la solution de tampon ajusté à 1 l par de l'eau déminéralisée.
- Le tampon Tricine 200 mM -diéthylènetriamine 20 mM (C) est préparé en dissolvant 35,84 g de Tricine dans environ 900 ml d'eau déminéralisée, puis en ajoutant 2,06 g de diéthylènetriamine (DETA). Le pH est ajusté à 9,40 à 22°C par environ 32,4 ml de NaOH 5M et le volume de la solution de tampon ajusté à 1 l par de l'eau déminéralisée.
- Le tampon Tricine 200 mM - N,N,N',N'-Tétraméthyl-1,4-butanediamine 8 mM (D) est préparé en dissolvant 35,84 g de Tricine dans environ 900 ml d'eau déminéralisée, puis en ajoutant 1,15 g de N,N,N',N'-Tétraméthyl-1,4-butanediamine. Le pH est ajusté à 9,19 à 22°C par environ 34,7 ml de NaOH 5M et le volume de la solution de tampon ajusté à 1 l par de l'eau déminéralisée.

### B) Echantillons cliniques :

Pour l'EC, le sang humain, décanté ou centrifugé, est dilué au 1/6^{ème} dans la solution hémolysante. Celle-ci est préparée en dissolvant 1,00 g de Triton X100, 2,50 g de saponine, 3,63 g de Tris dans environ 900 ml d'eau déminéralisée. Le pH est ajusté à 8,70 à 22, 0°C et le volume de la solution ajusté à 1 l par de l'eau déminéralisée. Les produits chimiques utilisés sont de grade analytique.

### Exemples 1à 5

Une solution de tampon d'analyse Tricine/DAB.2HCl est préparé comme ci-dessus.

L'électrophorèse a été réalisée selon la méthode ci-dessus sur un sang humain.

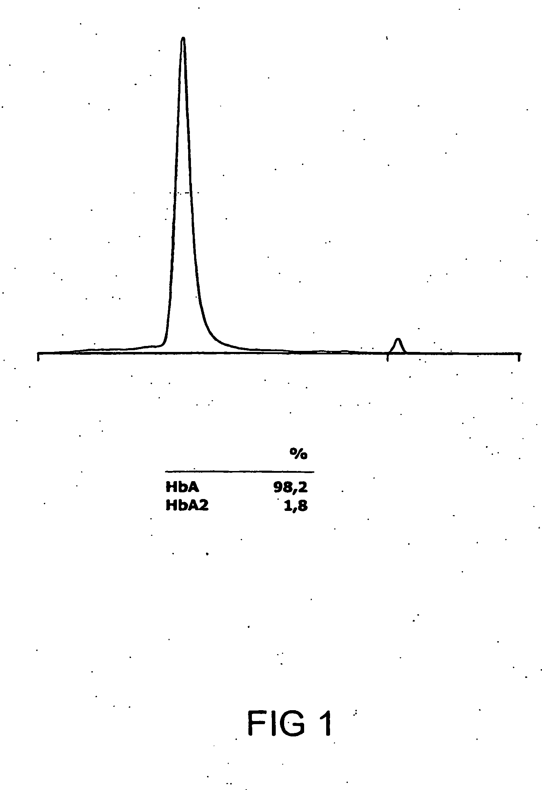

Exemple 1 : analyse d'un sang humain normal avec HbA en pic principal et HbA₂, fraction plus petite. (Figure 1)

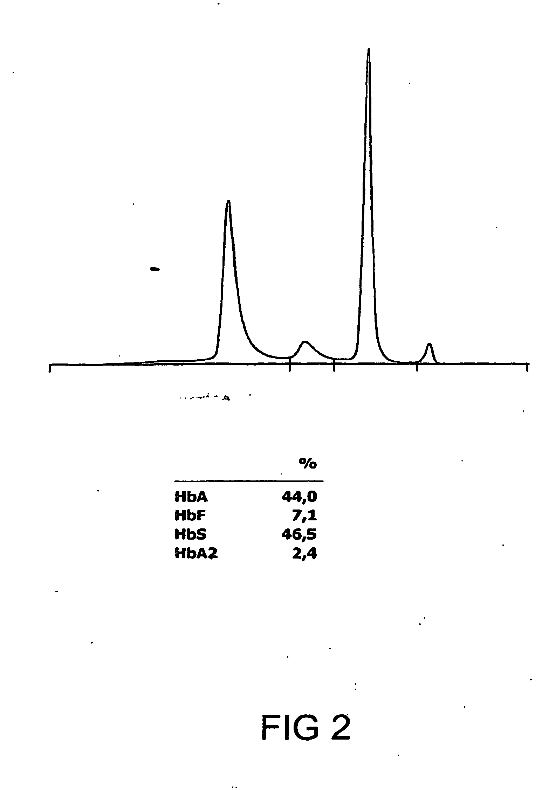

Exemple 2 : analyse d'un sang humain malade qui montre une fraction HbA plus faible, une fraction HbF augmentée, une fraction HbS forte et une fraction HbA₂ normale. Il s'agit d'une HbS hétérozygote à l'origine de la maladie de drépanocytose. (Figure 2)

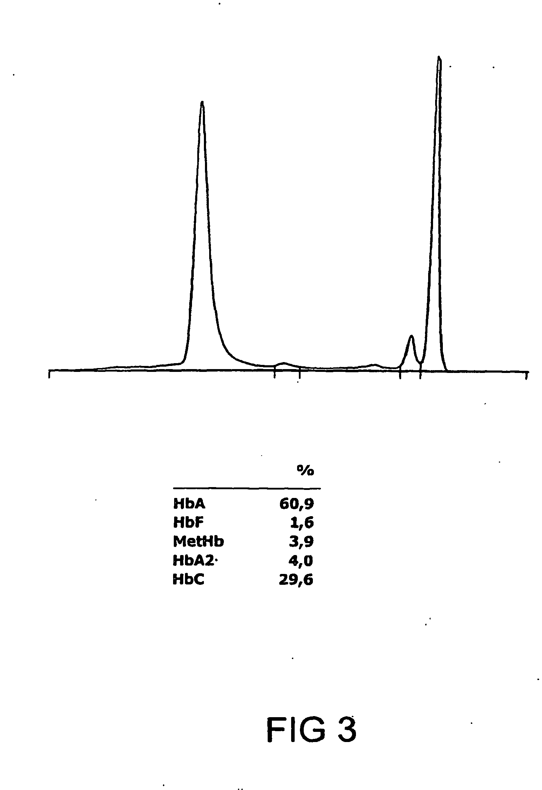

Exemple 3 : analyse d'un sang humain de malade présentant une hétérozygotie A/C. La fraction HbC sort juste avant la fraction HbA₂ dont elle est bien résolue. Cette bonne résolution autorise la quantification de A₂ ce qui permet d'orienter le diagnostic en cas de β-thalassémie, c'est-à-dire de fraction HbA₂ augmentée. (Figure 3)

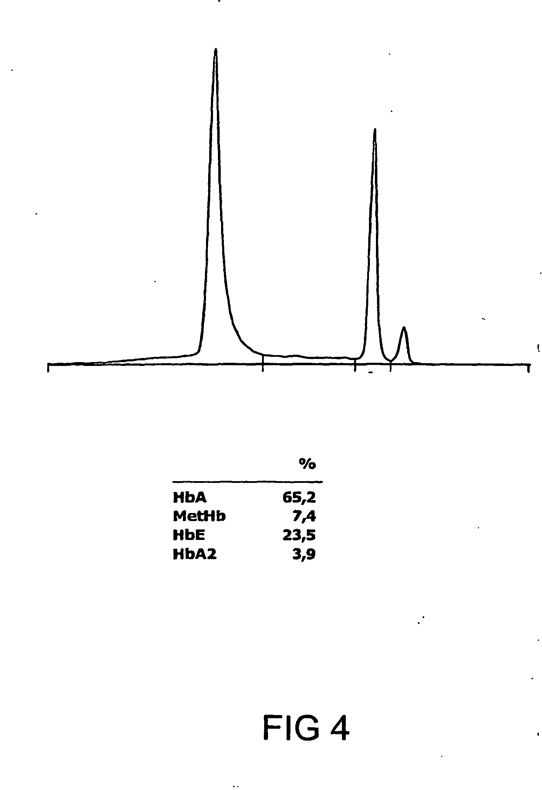

Exemple 4: analyse d'un sang humain présentant une hétérozygotie A/E. La fraction HbE sort juste après la fraction HbA₂ dont elle est totalement résolue. Il n'y a donc aucun problème pour quantifier HbA₂ en présence d'HbE. (Figure 4)

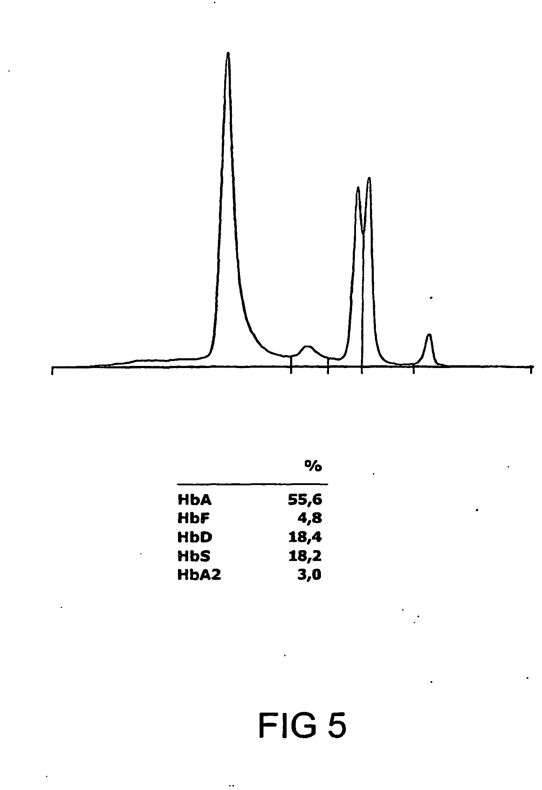

Exemple 5: analyse d'un mélange d'un sang présentant une hétérozygotie A/S et d'un sang présentant une hétérogygotie A/D Los Angeles. Les fractions HbS et HbD sont partiellement résolues ce qui permet de les distinguer l'une de l'autre sans nécessité d'une analyse complémentaire. (Figure 5)

Exemple 6 : analyse d'un sang humain β-thalassémique, c'est-à-dire présentant un pourcentage de la fonction HbA₂ augmenté et présentant une petite fraction HbF.

Quatre solutions de tampons d'analyse A, B, C et D sont préparées comme ci-dessus.

L'électrophorèse a été réalisée selon la méthode décrite ci-dessus.

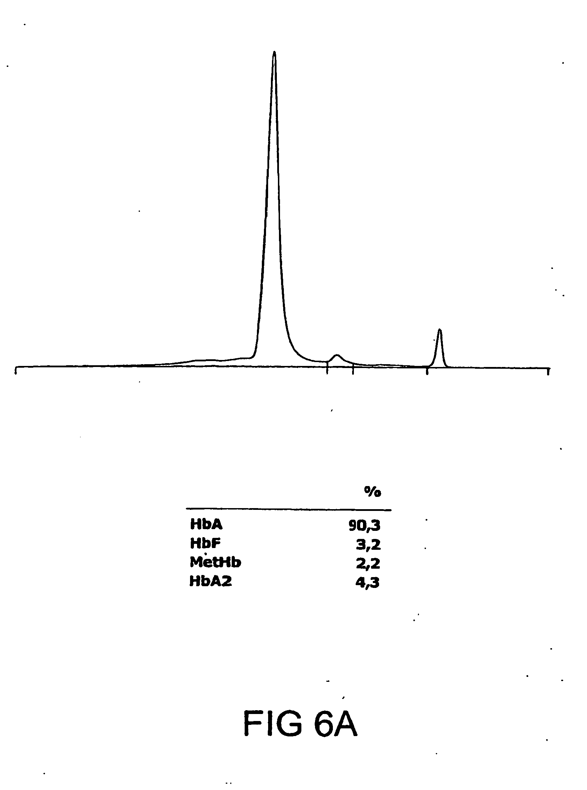

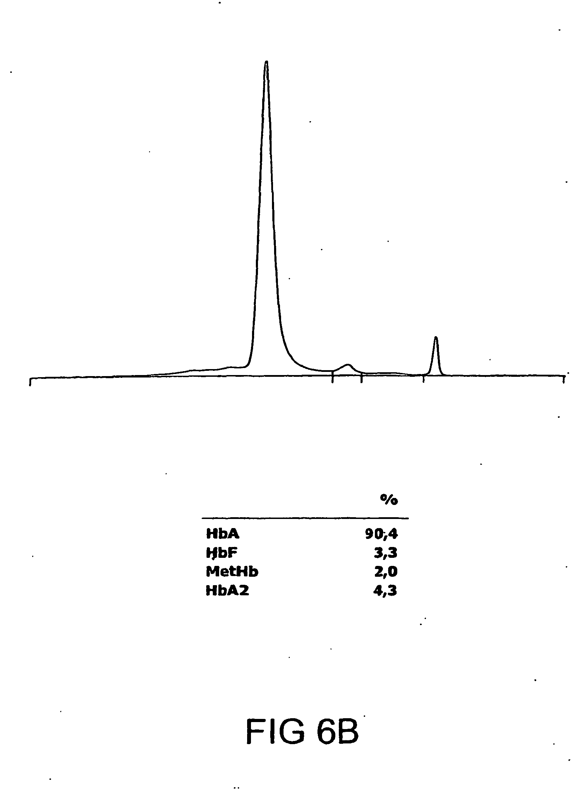

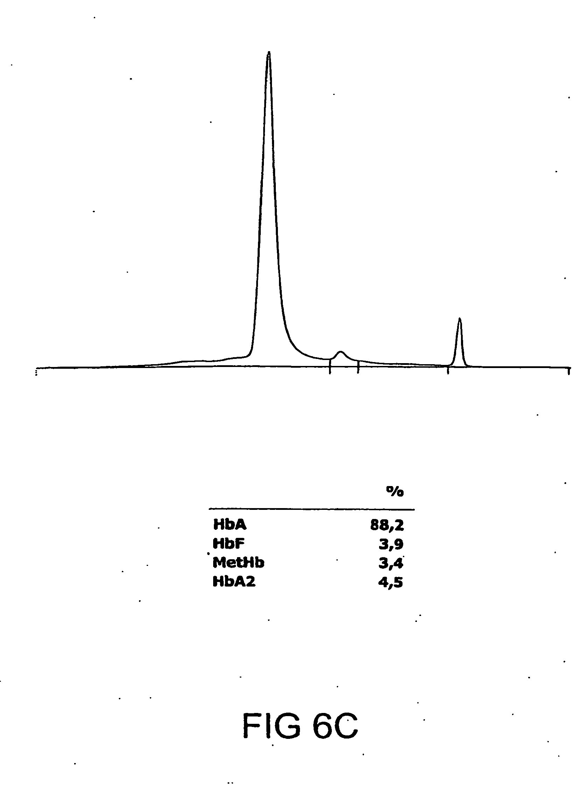

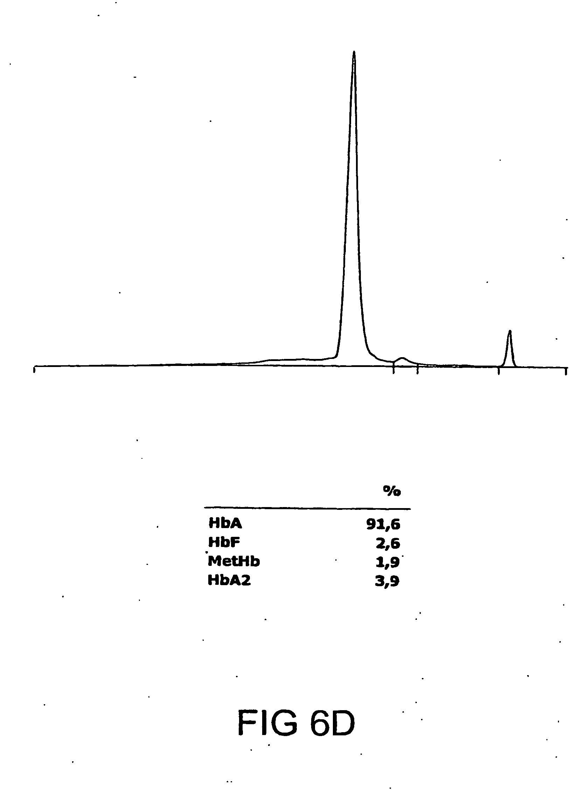

La figure 6a) montre le résultat avec le tampon A (Tricine/DAB.2HCl). La figure 6b) montre le résultat avec le tampon B (Tricine/1,5-diaminopentane.2HCl). La figure 6c) montre le résultat avec le tampon C (Tricine/DETA). La figure 6d) montre le résultat avec le tampon D (Tricine/N,N,N',N'-Tétraméthyl-1,4-butanediamine).

La comparaison de ces quatre figures permet d'observer, dans tous les cas, une bonne séparation de chacune des fractions ainsi qu'une bonne focalisation des petites fractions HbF et HbA₂.

Exemple 7: analyse d'un sang humain présentant une hétérozygotie drépanocytaire (HbS/HbA).

Quatre solutions de tampons d'analyse (A : Tricine/DAB.2HCl ; B : Tricine/1,5-diaminopentane.2HCl : C : Tricine/DETA ; D : Tricine/N,N,N',N'-Tétraméthyl-1,4-butanediamine) sont préparées comme ci-dessus.

L'électrophorèse a été réalisée selon la méthode décrite ci-dessus.

L'ordre des figures correspond aux tampons déjà évoqués. Là encore, on peut observer des profils comparables quel que soit le tampon utilisé.

### Références

1. Noriaki Ishioka et al., Biomedical Chromatography, vol. 6, 224-226 (1992).
2. Ahmet Sahin et al., Journal of Chromatography A, 709 (1995) 121-125.
3. Margaret A. Jenkins, Michael D. Guerin, Journal of Chromatography B, 682 (1996) 23-34.
4. Margaret A. Jenkins, Jean Hendy, lan L. Smith, J. CAP. ELEC. 004 : 3 (1997) 137-143.
5. Margaret A. Jenkins, Sujiva Ratnaike, Clinica Chimica Acta 289 (1999) 121-132.
6. Zak K. Shihabi et al., Electrophoresis, 21 (2000), 749-752.
7. James M. Hempe, Randall D. Craver, Electrophoresis, 21 (2000) 743-748.

## Revendications

1. Procédé de séparation des hémoglobines dans des échantillons biologiques par électrophorèse capillaire en solution libre (FSCE), procédé dans lequel on fait passer l'échantillon comportant lesdites hémoglobines dans un capillaire contenant un tampon d'analyse, comportant au moins une étape où on introduit l'échantillon dans un tube capillaire contenant une solution d'au moins un tampon d'analyse, **caractérisé en ce que** les hémoglobines sont l'hémoglobine A₂ et les hémoglobines C, D, E, S, F et/ou A, et **en ce que** le tampon est du type zwitterionique et est associé à au moins un ralentisseur de flux choisi, parmi les diamines aliphatiques, les polyamines aliphatiques, leurs dérivés et sels acceptables; et leurs mélanges.

2. Procédé selon la revendication 1, **caractérisé en ce qu'**il comporte en outre la séparation des hémoglobines par migration et la détection de ces hémoglobines.

3. Procédé selon la revendication 1 ou 2, **caractérisé en ce que** l'échantillon est un échantillon de sang, normal ou non, lavé, décanté, centrifugé ou total.

4. Procédé selon l'une des revendications 1 à 3, **caractérisé en ce que** l'échantillon est un échantillon de sang hémolysé.

5. Procédé selon l'une des revendications 1 à 4,
**caractérisé en ce que** le tapon zwitterionique est formé d'une ou deux molécules, et comporte au moins une fonction amine, au moins une fonction acide et au moins une fonction hydroxyle en position opposée à la fonction acide.

6. Procédé selon l'une des revendications 1 à 5, **caractérisé en ce que** le tampon zwitterionique est choisi parmi Tricine, TAPS, TABS, AMPSO, Bicine ou HEPBS, ou l'association de Tris, AMPD ou Bis Tris Propane avec un acide aminé.

7. Procédé selon l'une des revendications 1 à 6, **caractérisé en ce que** le tampon est la Tricine.

8. Procédé selon l'une des revendications 1 à 7, **caractérisé en ce que** ledit ralentisseur de flux est choisi parmi le 1,3-diaminopropane, le 1,4-diaminobutane, le 1,5-diaminopentane, le 1,6-diaminohexane, la diéthylènetriamine, la spermine, la N,N'-diméthyl-1,6-hexanediamine, la tétraéthylènepentamine, la N,N',N',N'-tétraméthyl-1,4-butanediamine et leurs dérivés ou sels acceptables, et leurs mélanges

9. Procédé selon l'une des revendications 1 à 8, **caractérisé en ce que** ledit ralentisseur de flux est choisi parmi le 1,4-diaminobutane, le 1,5-diaminopentane, le 1,6-diaminohexane, la diéthylènetriamine, la N,N,N',N'-tétraméthyl-1,4-butanediamine et leurs dérivés ou sels acceptables, et leurs mélanges.

10. Procédé selon l'une des revendications 1 à 9, **caractérisé en ce que** le ralentisseur de flux est le 1,4-diaminobutane.

11. Procédé selon l'une des revendications 1 à 10, **caractérisé en ce que** le ralentisseur de flux est le 1,4-diaminobutane sous forme chlorhydrate.

12. Procédé selon l'une des revendications 1 à 11, **caractérisé en ce que** la concentration en tampon dans la solution de tampon est comprise entre 20 mM et 500 mM.

13. Procédé selon l'une des revendications 1 à 12, **caractérisé en ce que** la concentration en tampon dans la solution de tampon est comprise entre 100 mM et 250 mM.

14. Procédé selon l'une des revendications 1 à 13, **caractérisé en ce que** la concentration en ralentisseur de flux dans la solution de tampon est comprise entre 0,01 et 50 mM.

15. Procédé selon l'une des revendications 1 à 14, **caractérisé en ce que** la concentration en ralentisseur de flux dans la solution de tampon est comprise entre 0,10 mM et 20 mM.

16. Procédé selon l'une des revendication 1 à 15, **caractérisé en ce que** la solution de tampon comporte en outre un modificateur de pH.

17. Procédé selon la revendication 16, **caractérisé en ce que** le modificateur de pH est choisi parmi l'hydroxyde de lithium, l'hydroxyde de sodium, l'hydroxyde de potassium, l'hydroxyde de rubidium, l'hydroxyde de césium, l'hydroxyde de francium, un hydroxyde de mono-, di-, tri- ou tétra-alkyl ammonium ayant de 1 à 8 atomes de carbone dans la partie alkyle.

18. Procédé selon l'une des revendications 1, à 17, **caractérisé en ce que** le capillaire est nu.

19. Procédé selon l'une des revendications 1 à 18, **caractérisé en ce que** le capillaire est en silice fondue.

20. Procédé selon l'une des revendications 1 à 19, **caractérisé en ce que** le pH de la solution de tampon d'analyse est compris entre 8 est 10.

21. Procédé selon l'une des revendications 1 à 20, **caractérisé en ce que** l'échantillon est préalablement dilué avec une solution de dilution appropriée, une solution hémolysante ou une solution de tampon d'analyse.

22. Utilisation d'un ralentisseur de flux choisi parmi les diamines aliphatiques, les polyamines aliphatiques, leurs dérivés et sels acceptables, et leurs mélanges, dans un procédé d'électrophorèse capillaire en solution libre, pour séparer des hémoglobines, en association avec au moins un tampon zwitterionique dans une solution de tampon, les hémoglobines étant l'hémoglobine A₂ et les hémoglobines C, D, E, S, F et/ou A.

23. Utilisation selon la revendication 22, dans laquelle le tampon zwitterionique et présent à une concentration d'environ 20 à 500 mM, et le ralentisseur est présent à une concentration d'environ 0,01 à 50 mM.

24. Trousse d'analyse de l'hémoglobine par électrophorèse capillaire en solution libre, comprenant au moins une solution de tampon d'analyse contenant (1) au moins un tampon d'analyse du type zwitterionique et (2) au moins un ralentisseur de flux parmi les diamines aliphatiques, les polyamines aliphatiques, leurs, dérivés et sels acceptables, et leurs mélanges.

25. Trousse d'analyse selon la revendication 24, **caractérisée en ce qu'**elle comporte en outre un modificateur de pH.

26. Trousse d'analyse selon la revendication 24 ou 25, **caractérisée en ce qu'**elle comporte en outre une solution hémolysante.

## Claims

1. A method for separating haemoglobins in biological samples by free solution capillary electrophoresis (FSCE), in which the sample comprising said haemoglobins is passed through a capillary containing an analysis buffer, comprising at least one step in which the sample is introduced into a capillary tube containing a solution of at least an analysis buffer,
**characterized in that** the haemoglobins are haemoglobin A₂ and C, D, E, S, F and/or A haemoglobins and **in that** the buffer is of the zwitterionic type and is associated with at least one flow inhibitor selected from aliphatic diamines, aliphatic polyamines and their acceptable derivatives and salts, and mixtures thereof.

2. A method according to claim 1, **characterized in that** it further comprises separating haemoglobins by migration and detecting said haemoglobins.

3. A method according to claim 1 or 2, **characterized in that** the sample is a sample of blood which may be normal or abnormal, washed, decanted, centrifuged or whole blood.

4. A method according to one of claims 1 to 3, **characterized in that** the sample is a sample of haemolyzed blood.

5. A method according to one of claims 1 to 4, **characterized in that** the zwitterionic buffer is formed by one or two molecules and comprises at least one amine function, at least one acid function and at least one hydroxyl function in the position opposite to the acid function.

6. A method according to one of claims 1 to 5, **characterized in that** the zwitterionic buffer is selected from tricine, TAPS, TABS, AMPSO, bicine or HEPBS, or an association of Tris, AMPD or bis Tris propane with an amino acid.

7. A method according to one of claims 1 to 6, **characterized in that** the buffer is tricine.

8. A method according to one of claims 1 to 7, **characterized in that** said flow inhibitor is selected from 1,3-diaminopropane, 1,4-diaminobutane, 1,5-diaminopentane, 1,6-diaminohexane, diethylenetriamine, spermine, N,N'-dimethyl-1,6-hexanediamine, tetraethylenepentamine, N,N,N',N'-tetramethyl-1,4-butanediamine and their acceptable derivatives and salts, and mixtures thereof.

9. A method according to one of claims 1 to 8, **characterized in that** said flow inhibitor is selected from 1,4-diaminobutane, 1,5-diaminopentane, 1,6-diaminohexane, diethylenetriamine and N,N,N',N'-tetramethyl-1,4-butanediamine, and their acceptable derivatives and salts, and mixtures thereof. .

10. A method according to one of claims 1 to 9, **characterized in that** the flow inhibitor is 1,4-diaminobutane.

11. A method according to one of claims 1 to 10, **characterized in that** the flow inhibitor is 1,4-diaminobutane in the hydrochloride form.

12. A method according to one of claims 1 to 11, **characterized in that** the concentration of buffer in the buffer solution is in the range 20 mM to 500 mM.

13. A method according to one of claims 1 to 12, **characterized in that** the concentration of buffer in the buffer solution is in the range 100 mM to 250 mM.

14. A method according to one of claims 1 to 13, **characterized in that** the concentration of flow inhibitor in the buffer solution is in the range 0.01 to 50 mM.

15. A method according to one of claims 1 to 14, **characterized in that** the concentration of flow inhibitor in the buffer solution is in the range 0.10 mM to 20 mM.

16. A method according to one of claims 1 to 15, **characterized in that** the buffer solution further comprises a pH modifier.

17. A method according to claim 16, **characterized in that** the pH modifier is selected from lithium hydroxide, sodium hydroxide, potassium hydroxide, rubidium hydroxide, caesium hydroxide, francium hydroxide, and mono-, di-, tri- or tetra- alkylammonium hydroxide containing 1 to 8 carbon atoms in the alkyl portion.

18. A method according to claim one of claims 1 to 17, **characterized in that** the capillary is naked.

19. A method according to one of claims 1 to 18, **characterized in that** the capillary is formed from fused silica.

20. A method according to one of claims 1 to 19, **characterized in that** the pH of the analysis buffer solution is in the range 8 to 10.

21. A method according to one of claims 1 to 20, wherein the sample is initially diluted with a suitable diluting solution, a haemolyzing solution or an analysis buffer solution.

22. Use of a flow inhibitor selected from aliphatic diamines, aliphatic polyamines and their acceptable derivatives and salts, and mixtures thereof, in a free solution capillary electrophoresis method for separating haemoglobins, in association with at least one zwitterionic buffer in a buffer solution, the haemoglobins being haemoglobin A₂ and C, D, E, S, F and/or A haemoglobins.

23. Use according to claim 22, in which the zwitterionic buffer is present in a concentration of about 20 to 500 mM, and the inhibitor is present in a concentration of about 0.01 to 50 mM.

24. A free solution capillary electrophoresis haemoglobin analysis kit comprising at least one analysis buffer containing (1) at least one zwitterionic type analysis buffer and (2) at least one flow inhibitor is selected from aliphatic diamines, aliphatic polyamines and their acceptable derivatives and salts, and mixtures thereof.

25. An analysis kit according to claim 24, **characterized in that** it further comprises a pH modifier.

26. An analysis kit according to claim 24 or 25, **characterized in that** it further comprises a haemolyzing solution.

## Patentansprüche

1. Verfahren zum Abtrennen von Hämoglobinen in biologischen Proben durch Kapillarelektrophorese in freier Lösung (FSCE), wobei in dem Verfahren eine Probe, die die Hämoglobine umfasst, in eine Kapillare geleitet wird, die einen Analysepuffer enthält, umfassend mindestens einen Schritt, in dem die Probe in ein Kapillarröhrchen eingeführt wird, das eine Lösung von mindestens einem Analysepuffer enthält,
**dadurch gekennzeichnet, dass** die Hämoglobine das Hämoglobin A₂ und die Hämoglobine C, D, E, S, F und/oder A sind, und dadurch, dass der Puffer vom Zwitteriontyp ist und mit mindestens einem Durchflussverzögerer kombiniert ist, der ausgewählt ist aus aliphatischen Diaminen, aliphatischen Polyaminen, Derivaten und akzeptablen Salzen davon und Mischungen davon.

2. Verfahren nach Anspruch 1, **dadurch gekennzeichnet, dass** es weiter die Abtrennung von Hämoglobinen durch Migration und die Detektion dieser Hämoglobine umfasst.

3. Verfahren nach Anspruch 1 oder 2, **dadurch gekennzeichnet, dass** die Probe eine Blutprobe, Normalprobe oder nicht Normalprobe, gewaschen, dekantiert, zentrifugiert oder vollständig ist.

4. Verfahren nach einem der Ansprüche 1 bis 3, **dadurch gekennzeichnet, dass** die Probe eine hämolysierte Blutprobe ist.

5. Verfahren nach einem der Ansprüche 1 bis 4, **dadurch gekennzeichnet, dass** der zwitterionische Puffer aus einem oder zwei Molekülen gebildet ist und mindestens eine Aminfunktion, mindestens eine Säurefunktion und mindestens eine Hydroxylfunktion in einer der Säurefunktion gegenüberliegenden Position umfasst.

6. Verfahren nach einem der Ansprüche 1 bis 5, **dadurch gekennzeichnet, dass** der zwitterionische Puffer ausgewählt ist aus Tricin, TAPS, TABS, AMPSO, Bicin oder HEPBS oder einer Kombination von Tris, AMPD oder Bis-Tris-Propan mit einer Aminosäure.

7. Verfahren nach einem der Ansprüche 1 bis 6, **dadurch gekennzeichnet, dass** der Puffer Tricin ist.

8. Verfahren nach einem der Ansprüche 1 bis 7, **dadurch gekennzeichnet, dass** der Durchflussverzögerer ausgewählt ist aus 1,3-Diaminopropan, 1,4-Diaminobutan, 1,5-Diaminopentan, 1,6-Diaminohexan, Diethylentriamin, Spermin, N-N'-Dimethyl-1,6-hexandiamin, Tetraethylenpentamin, N,N,N',N'-Tetramethyl-1,4-butandiamin und Derivaten oder akzeptablen Salzen davon und Mischungen davon.

9. Verfahren nach einem der Ansprüche 1 bis 8, **dadurch gekennzeichnet, dass** der Durchflussverzögerer ausgewählt ist aus 1,4-Diaminobutan, 1,5-Diaminopentan, 1,6-Diaminohexan, Diethylentriamin, N,N,N',N'-Tetramethyl-1,4-butandiamin und Derivaten oder akzeptablen Salzen davon und Mischungen davon.

10. Verfahren nach einem der Ansprüche 1 bis 9, **dadurch gekennzeichnet, dass** der Durchflussverzögerer 1,4-Diaminobutan ist.

11. Verfahren nach einem der Ansprüche 1 bis 10, **dadurch gekennzeichnet, dass** der Durchflussverzögerer 1,4-Diaminobutan in Form seines Chlorhydrats ist.

12. Verfahren nach einem der Ansprüche 1 bis 11, **dadurch gekennzeichnet, dass** die Konzentration an Puffer in der Pufferlösung zwischen 20 mM und 500 mM beträgt.

13. Verfahren nach einem der Ansprüche 1 bis 12, **dadurch gekennzeichnet, dass** die Konzentration an Puffer in der Pufferlösung zwischen 100 mM und 250 mM beträgt.

14. Verfahren nach einem der Ansprüche 1 bis 13, **dadurch gekennzeichnet, dass** die Konzentration an Durchflussverzögerer in der Pufferlösung zwischen 0,01 mM und 50 mM beträgt.

15. Verfahren nach einem der Ansprüche 1 bis 14, **dadurch gekennzeichnet, dass** die Konzentration an Durchflussverzögerer in der Pufferlösung zwischen 0,10 mM und 20 mM beträgt.

16. Verfahren nach einem der Ansprüche 1 bis 15, **dadurch gekennzeichnet, dass** die Pufferlösung weiter einen pH-Einsteller umfasst.

17. Verfahren nach Anspruch 16, **dadurch gekennzeichnet, dass** der pH-Einsteller ausgewählt ist aus Lithiumhydroxid, Natriumhydroxid, Kaliumhydroxid, Rubidiumhydroxid, Cäsiumhydroxid, Franciumhydroxid, einem Mono-, Di-, Tri- oder Tetraalkylammoniumhydroxid mit 1 bis 8 Kohlenstoffatomen im Alkylrest.

18. Verfahren nach einem der Ansprüche 1 bis 17, **dadurch gekennzeichnet, dass** die Kapillare nackt ist.

19. Verfahren nach einem der Ansprüche 1 bis 18, **dadurch gekennzeichnet, dass** die Kapillare aus glasartigem Siliciumoxid gebildet ist.

20. Verfahren nach einem der Ansprüche 1 bis 19, **dadurch gekennzeichnet, dass** der pH der Analysepufferlösung zwischen 8 und 10 beträgt.

21. Verfahren nach einem der Ansprüche 1 bis 20, **dadurch gekennzeichnet, dass** die Probe zuvor mit einer geeigneten Verdünnungslösung, hämolysierenden Lösung oder einer Analysepufferlösung verdünnt wird.

22. Verwendung eines Durchflussverzögerers, der ausgewählt ist aus aliphatischen Diaminen, aliphatischen Polyaminen, Derivaten und akzeptablen Salzen davon und Mischungen davon, in einem Verfahren zur Kapillarelektrophorese in freier Lösung zum Abtrennen von Hämoglobinen in Kombination mit mindestens einem zwitterionischen Puffer in einer Pufferlösung, wobei die Hämoglobine das Hämoglobin A₂ und die Hämoglobine C, D, E, S, F und/oder A sind.

23. Verwendung nach Anspruch 22, bei der der zwitterionische Puffer in einer Konzentration von ungefähr 20 bis 500 mM vorhanden ist und der Durchflussverzögerer in einer Konzentration von ungefähr 0,01 bis 50 mM vorhanden ist.

24. Kit zur Analyse von Hämoglobin durch Kapillarelektrophorese in freier Lösung, umfassend mindestens eine Analysepufferlösung, die enthält:
(1) mindestens einen Analysepuffer vom Zwitteriontyp und
(2) mindestens einen Durchflussverzögerer aus aliphatischen Diaminen, aliphatischen Polyaminen, Derivaten und akzeptablen Salzen davon und Mischungen davon.

25. Analysekit nach Anspruch 24, **dadurch gekennzeichnet, dass** er weiter einen pH-Einsteller umfasst.

26. Analysekit nach Anspruch 24 oder 25, **dadurch gekennzeichnet, dass** er weiter eine hämolysierende Lösung umfasst.
